# EUROPEAN PATENT SPECIFICATION

(11) **EP 3 429 377 B1**
(45) Date of publication and mention of the grant of the patent: **09.09.2020**
(21) Application number: 17727684.7
(22) Date of filing: 15.03.2017
(51) Int. Cl.: A23L 29/00, A23P 10/28, A23P 10/47, A23L 33/115, A23L 33/12, A61K 9/16, A61K 31/202, A61K 35/60, A61K 9/20, A61K 9/28, A61K 35/618, A61K 36/02

(54) **POWDERS AND TABLETS COMPRISING OMEGA-3 FATTY ACID DERIVATIVES AND METHOD FOR THEIR PRODUCTION**
PULVER UND TABLETTEN AUS OMEGA-3 FETTSÄURE-DERIVATEN UND VERFAHREN ZU DEREN HERSTELLUNG
POUDRES ET COMPRIMÉS COMPRENANT DES DÉRIVÉS D'ACIDES GRAS OMÉGA-3 ET LEUR PROCÉDÉ DE PRODUCTION

(30) Priority: 16.03.2016 US 201662309013 P
(43) Date of publication of application: 23.01.2019
(73) Proprietor: Golden Omega Norway AS, 0422 Oslo (NO)
(72) Inventor: LIEN VESTLAND, Tina, 0422 Oslo (NO); KLAVENESS, Jo, 0422 Oslo (NO)
(74) Representative: Algemeen Octrooi- en Merkenbureau B.V.
(86) International application number: PCT/IB2017/000548
(87) International publication number: WO 2017/158439

(56) References cited:
- WO-A2-2008/146016
- US-A1- 2012 156 296
- SZENTE L ET AL: "FATTY ACID-CYCLODEXTRIN COMPLEXES: PROPERTIES AND APPLICATIONS", JOURNAL OF INCLUSION PHENOMENA AND MOLECULAR RECOGNITION IN CHEMISTRY, KLUWER, DORDRECHT, NL, vol. 16, no. 4, 1 January 1993 (1993-01-01), pages 339-354, XP000675556, ISSN: 0923-0750, DOI: 10.1007/BF00708714

## Description

### FIELD OF THE INVENTION

The present invention relates to an improved method for preparation of powder and tablets comprising omega-3 fatty acid derivatives and beta-cyclodextrin.

### BACKGROUND OF THE INVENTION

Omega-3 comprising products are generally provided either in the form of oil encapsulated in soft capsules or in the form of free oil (cod liver oil products). There has been a need for omega-3 products with different and improved properties relative to omega-3 in the form of oil. These improved properties are one or more of the following: improved oxidative stability, reduced fish taste typically from gastrointestinal reflux, efficient uptake from the gastrointestinal system, improved technical possibilities to prepare stable combination products (products comprising omega-3 plus one or more active components like minerals, vitamins, drug substances or food additives) and finally omega-3 products that can be used in food products like drinks (e.g. juice) and semisolid/solid food products (e.g. yoghurt and bread). Various forms of dry powders based on encapsulation of omega-3 droplets have been developed and these dry powders are extensively used in various drug products. These powders based on physical encapsulation of omega-3 oil do not have all the properties listed above and cannot be tableted due to the high pressure and increased temperature during tableting. WO 2008/146016 A2 discloses a pharmaceutical or nutraceutical tablet for oral administration comprising at least two fatty acids or derivatives thereof at a high dosage level and cyclodextrin.

### SUMMARY OF THE INVENTION

The present invention relates to an improved method for preparation of powder and tablets comprising omega-3 fatty acid derivatives and beta-cyclodextrin. The claimed invention is strictly defined in the appended set of claims.

Accordingly, the present invention provides compositions comprising a dry powder comprising beta-cyclodextrin in an amount of from 60% to 90% w/w of the powder and a lipid component in an amount of from about 10% to 40% w/w of the powder, wherein the lipid component is characterized as having a surfactant content of from about 10% to 35% w/w of the lipid component, as claimed.

The lipid component comprises an omega-3 fatty acid or derivative thereof selected from the group consisting of omega-3 triglycerides. In some embodiments, the omega-3 fatty acids or derivatives thereof have an EPA:DHA ratio of greater than 1:1. In some embodiments, the omega-3 fatty acids or derivatives thereof have a DHA:EPA ratio of greater than 1:1. In some embodiments, the omega-3 fatty acid or derivative thereof selected from the group consisting of omega-3 triglycerides comprises EPA and DHA fatty acids at a concentration of from 10% to 70% w/w of the fatty acids in the omega-3 triglycerides. In some embodiments, the omega-3 fatty acid or derivative thereof selected from the group consisting of omega-3 triglycerides comprises EPA and DHA fatty acids at a concentration of from 30% to 60% w/w of the fatty acids in the omega-3 triglycerides.

The surfactant is a diglyceride of fatty acids. In some embodiments, the diglycerides of fatty acids comprise a mixture of diglyceride compounds wherein the fatty acid components of the diglycerides compounds are selected from saturated, monounsaturated and polyunsaturated fatty acids. In some embodiments, the polyunsaturated fatty acids are omega-3 fatty acids. In some embodiments, the omega-3 fatty acids are selected from EPA and DHA. The concentration of the surfactant in the lipid component is from 10% to 35% w/w of the lipid component.

In some embodiments, the powder composition is spray granulated. In some embodiments, the powder composition is spray granulated and has a particle size distribution of 50-650 microns. In some embodiments, the powder composition is spray granulated and has a particle size distribution of 200-500 microns.

Furthermore, the present invention provides tableted lipid formulations comprising beta-cyclodextrin in a concentration of from 60% to 90% w/w of the tablet and a lipid component in a concentration of from 10% to 40% w/w of the tablet, wherein the lipid component is characterized as having a surfactant content of from 10% to 35% w/w of the lipid component, as claimed.

In some embodiments, the tablet has a crushing strength of greater than 5 kN. In some embodiments, the tablet has a crushing strength of greater than 7 kN. In some embodiments, the tablet has a crushing strength of from 5 to 10 kN.

In some embodiments, the present invention provides tableted lipid formulations comprising beta-cyclodextrin in a concentration of from 60% to 90% w/w of the tablet and a lipid component in a concentration of from 10% to 40% w/w of the tablet, wherein the lipid component is characterized as having a diglyceride content of from 10% to 35% w/w of the lipid component, wherein the tablet has a crushing strength of greater than 3 kN.

In some embodiments, the tablet has a crushing strength of greater than 5 kN. In some embodiments, the tablet has a crushing strength of greater than 7 kN. In some embodiments, the tablet has a crushing strength of from 5 to 10 kN.

In some embodiments, the omega-3 fatty acids or derivatives thereof have an EPA:DHA ratio of greater than 1:1. In some embodiments, the omega-3 fatty acids or derivatives thereof have a DHA:EPA ratio of greater than 1:1. In some embodiments, the omega-3 triglycerides are a marine oil. In some embodiments, the marine oil is selected from the group consisting of fish oil, squid oil and algal oil. In some embodiments, the omega-3 fatty acid or derivative thereof selected from the group consisting of omega-3 triglycerides comprises EPA and DHA fatty acids at a concentration of from 10% to 70% w/w of the fatty acids in the omega-3 triglycerides. In some embodiments, the omega-3 fatty acid or derivative thereof selected from the group consisting of omega-3 triglycerides comprises EPA and DHA fatty acids at a concentration of from 30% to 60% w/w of the fatty acids in the omega-3 triglycerides.

In some embodiments, the diglycerides comprise a mixture of diglyceride compounds wherein the fatty acid components of the diglycerides compounds are selected from saturated, monounsaturated and polyunsaturated fatty acids. In some embodiments, the polyunsaturated fatty acids are omega-3 fatty acids. In some embodiments, omega-3 fatty acids are selected from EPA and DHA.

In some embodiments, the tableted formulation is coated. In some embodiments, the tableted formulation is coated with an agent selected from the group consisting of polyvinyl acetate, methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, cellulose acetate trimellitate, and sodium alginate.

In some embodiments, the lipid component is combined with an additional nutraceutical agent that is not an omega-3 fatty acid or derivative thereof. In some embodiments, the lipid component is combined with an additional pharmaceutical agent that is not an omega-3 fatty acid or derivative thereof.

Furthermore, the present invention provides processes for making a tabletable lipid powder comprising: combining an aqueous solution of beta-cyclodextrin with a lipid component in an amount of from 10% to 40% w/w of the beta-cyclodextrin the solution, wherein the lipid component comprises one or more surfactants at a concentration of from 10% to 35% w/w of the lipid component; mixing the aqueous solution of beta-cyclodextrin and the lipid component to provide a mixture; and removing water from the mixture to provide a dry powder, as claimed.

In some embodiments, the omega-3 fatty acids or derivatives thereof have an EPA:DHA ratio of greater than 1:1. In some embodiments, the omega-3 fatty acids or derivatives thereof have a DHA:EPA ratio of greater than 1:1. In some embodiments, the omega-3 triglycerides are a marine oil. In some embodiments, the marine oil is selected from the group consisting of fish oil, squid oil and algal oil. In some embodiments, the omega-3 fatty acid or derivative thereof selected from the group consisting of omega-3 triglycerides comprises EPA and DHA fatty acids at a concentration of from 10% to 70% w/w of the fatty acids in the omega-3 triglycerides. In some embodiments, the omega-3 fatty acid or derivative thereof selected from the group consisting of omega-3 triglycerides comprises EPA and DHA fatty acids at a concentration of from 20% to 45% w/w of the fatty acids in the omega-3 triglycerides.

The surfactant is a diglyceride of fatty acids. In some embodiments, the diglycerides of fatty acids comprise a mixture of diglyceride compounds wherein the fatty acid components of the diglycerides compounds are selected from saturated, monounsaturated and polyunsaturated fatty acids. In some embodiments, the polyunsaturated fatty acids are omega-3 fatty acids. In some embodiments, the omega-3 fatty acids are selected from EPA and DHA.

In some embodiments, the removing water from the mixture to provide a dry powder further comprises spray drying. In some embodiments, the removal of water is performed as spray granulation and the powder has a particle size distribution of 50-650 microns. In some embodiments, the removal of water is performed as spray granulation and the powder has a particle size distribution of 200-500 microns.

In some embodiments, the processes further comprise the step of forming a tablet from the dry powder. In some embodiments, the tablet has a crushing strength of greater than 5 kN. In some embodiments, the tablet has a crushing strength of greater than 7 kN. In some embodiments, the tablet has a crushing strength of from 5 to 10 kN.

### DETAILED DESCRIPTION OF THE INVENTION

The present invention relates to the method to use diglycerides, in the preparation process for powders comprising beta-cyclodextrin and omega-3 fatty acids and derivatives thereof and to the dry powders and tablets comprising diglycerides.

The inventors unexpectedly observed that the addition of between 10% and 35% diglycerides to an omega-3 oil composition (% weight/weight (w/w) calculated as weight of diglycerides divided by the total weight of the diglycerides plus the weight of the oil) allowed for the production of a beta-cyclodextrin powder with superior tabletting properties as well as tablets with superior properties.

The most preferred diglycerides for use in the present invention comprise a mixture of diglyceride compounds, where the fatty acid components in the mixture of diglyceride molecules can be saturated, monounsaturated and/or polyunsaturated, including omega-3 fatty acids like EPA and DHA.

The diglyceride molecules typically comprise saturated, monounsaturated and/or polyunsaturated fatty acids of various number of carbon atoms and various number of double bonds. Some typical fatty acids include fatty acids belonging to one or more of the following groups of fatty acids: 14:0, 15:0, 16:0, 16: 1, 17:0, 18:0, 18: 1, 18:2, 18 :3, 18: 4, 20: 1, 20: 4, 20:5, 22: 1, 22:5 and 22:6. The first number represents the number of carbon atoms and the last number represents the number of double bonds. EPA belongs to the fatty acid group 20:5 with 20 carbon atoms and 5 double bonds, while DHA belongs to the fatty acid group 22:6 with 22 carbon atoms and 6 double bonds. Both EPA and DHA have cis (Z)-isomer double bonds.

The diglycerides described according to the present invention might be naturally present in the oil or present in the oil as a result of the production process. The diglyceride might also be added to the oil before processing to a tablettable powder. In any event, the diglyceride content of the oil is adjusted to be in the range of from 10% to 35% of the weight of the oil on a w/w basis.

The diglyceride can be in the form of 1,2-diacylglycerols and/or 1,3-diacylglycerols. Diglycerides of fatty acids are approved food additives as emulsifiers. The diglycerides preferably have an HLB of about 3.

One of the most preferred aspects of the present invention is to use oil comprising a mixture of diglycerides where one or more of the single diglyceride molecular components are diglycerides with one or two EPA fatty acids or diglycerides with one or two DHA fatty acids.

If one component in the diglyceride mixture is a diglyceride with EPA, the other fatty acid might typically be an acid selected among the following groups of fatty acids: 14:0, 15:0, 16:0, 16: 1, 17:0, 18:0, 18: 1, 18:2, 18 :3, 18: 4, 20: 1, 20: 4, 20:5, 22: 1, 22:5 and 22:6.

If one component in the diglyceride mixture is a diglyceride with DHA, the other fatty acid might typically be an acid selected among the following groups of fatty acids: 14:0, 15:0, 16:0, 16: 1, 17:0, 18:0, 18: 1, 18:2, 18 :3, 18: 4, 20: 1, 20: 4, 20:5, 22: 1, 22:5 and 22:6.

The inclusion of such diglycerides improve(s) the quality of the powder both with regard to stability and tabletability. The present invention therefor relates to methods for preparation of powder and tablets comprising omega-3 fatty acid derivatives and beta-cyclodextrin and products prepared by the method. The method is characterized by including of one or more diglycerides during preparation of the aqueous slurry before preparation of the dry powder. The surfactant will generally be present in the powder and thereby the tablets. Aspects of the present invention are therefore powders and tablets comprising omega-3 fatty acid derivatives, beta-cyclodextrin and one or more diglycerides.

The method to use diglycerides in the preparation process for powders comprising beta-cyclodextrin and omega-3 is new and the obtained powders showed unexpected improved properties. Dry powders and tablets comprising omega-3 fatty acids and derivatives thereof, beta-cyclodextrin, and diglycerides are also new.

The description below describes the following aspects of the present invention: (1) Method; (2) Powder prepared according to the method; and (3) Tablets comprising omega-3, cyclodextrin and diglycerides.

### 1. Method

One aspect of the present invention relates to a method for preparation of dry powder comprising omega-3, beta-cyclodextrin using diglycerides. A preferred aspect of the method is a method for preparation of dry powder comprising omega-3, beta-cyclodextrin using diglycerides, where the dry powder is a tabletable powder. A more preferred aspect of this aspect of method is a method for preparation of dry powder comprising omega-3, beta-cyclodextrin using diglycerides, where the dry powder is a tabletable powder that can be tableted using standard tableting equipment producing more than 10,000 tablets per hour and the tablets can be prepared continuously for hours.

In preferred embodiments, beta-cyclodextrin, one or more omega-3 fatty acid and derivatives thereof, and diglycerides, are combined in an aqueous mixture. The mixture is agitated, for example by stirring, for a period of from about 5 minutes to 300 minutes, preferably from about 30 to about 90 minutes, and most preferably for about 60 minutes. The water is then removed from the mixture, for example by evaporation under reduced pressure to yield a dry, tabletable powder. In some embodiments, the water is preferably removed by spray drying or granulation. In some embodiments, the powder has a particle size distribution of 50-650 microns, and most preferably from 200-500 microns, following spray drying or granulation.

From 10% to 40%, 20% to 40%, 30% to 40%, 10% to 35%, 20% to 35%, 25% to 35%, 30% to 35%, at least 10%, at least 20%, at 30% or at least 35% w/w of an oil component is combined with the beta-cyclodextrin, wherein w/w refers to the total weight of the oil component to the total weight of beta-cyclodextrin. The w/w% above includes the weight of diglycerides in the oil used according to the present invention. In some embodiments, the oil component preferably comprises one or more omega-3 fatty acids or derivatives thereof. In some embodiments, the one or more omega-3 fatty acids or derivatives thereof are selected from omega-3 triglycerides, alone or in combination. In some embodiments, one or more of these omega 3 fatty acids and derivatives thereof are the main omega-3 components used to prepare the dry powder. In some embodiments, one or more of these omega 3 fatty acids and derivatives thereof are the main oil components used to prepare the dry powder (e.g., the oil component used in the process comprises greater than about 60%, 70%, 80%, w/w of the specified omega-3 fatty acid or derivative thereof wherein w/w refers to the total weight of the specified omega-3 fatty acid or derivative thereof per the total weight of the oil component). In some embodiments, one or more of these omega 3 fatty acids and derivatives thereof are from a marine source, such as fish, algae, or have been prepared from raw products from fish or algae. In some embodiments, one or more of these omega 3 fatty acids and derivatives thereof are from plants or vegetables or have been prepared from raw products from plants and vegetables.

In some embodiments, the omega-3 fatty acids or derivatives are preferably selected from EPA and DHA and combinations thereof. In some embodiments, the omega 3 fatty acids and derivatives thereof comprise more EPA than DHA. In some embodiments, the omega 3 fatty acids and derivatives thereof comprise more DHA than EPA. In some embodiments, the omega 3 fatty acids and derivatives thereof are enriched for EPA, e.g., more than 90% w/w of the total omega 3 fatty acids and derivatives thereof in the powder is EPA where the w/w% is the weight of EPA per total weight of fatty acids in the powder. In some embodiments, the omega 3 fatty acids and derivatives thereof are enriched for DHA, e.g., more than 90% w/w of the total omega 3 fatty acids and derivatives thereof in the powder is DHA where the w/w% is the weight of DHA per total weight of fatty acids in the powder. In some embodiments, the lipid component preferably comprises from about 30% to 60% w/w EPA and/or DHA.

From 10% to 30% w/w, 15% to 30% w/w, 18% to 30% w/w, 20% to 30% w/w surfactant or combination of surfactants is included with the oil component, wherein w/w refers to the total weight of surfactant (or combination thereof) to the total weight of the oil component.

The surfactants are diglycerides and the most preferred surfactants are diglycerides where one or more of the acids are omega-3 fatty acids. In especially preferred embodiments, the diglycerides comprise of a mixture of diglyceride compounds where the fatty acid components in the mixture of diglyceride molecules can be saturated, monounsaturated and polyunsaturated including omega-3 fatty acids like EPA and DHA.

A person skilled in the art would expect based on the unexpectedly good results with omega-3 oils comprising diglyceride for preparation of tablettable powder and tablets that other surfactants or surfactant mixtures with similar HLB-values will be as helpful as diglycerides.

In some embodiments, additional active ingredients may be included in the lipid component along with omega-3 fatty acids or derivatives thereof. Suitable additional active ingredients include, but are not limited to other active fatty acids such as omega-6 fatty acids, conjugated fatty acids such as conjugated linoleic acid fatty acid, and lipophilic drugs such as as Class II and Class IV drugs as classified under the Biopharmaceutics Classification System. Indeed, a variety a nutraceutical and pharmaceutical agents may be included in the lipid component. In some preferred embodiments, the nutraceutical and pharmaceutical agents are lipophilic.

In some embodiments, the active ingredient is a pharmaceutical ingredient selected from the groups consisting of antineoplastic, antifungal, antiviral, anticonvulsant, antiepileptic, immunosuppressant, and erectile dysfunction drugs. The BCS is a guide for predicting the intestinal drug absorption provided by the U.S. Food and Drug Administration. This system restricts the prediction using the parameters solubility and intestinal permeability. According to the Biopharmaceutics Classification System, drug substances are classified as follows: Class I -high permeability, high solubility (compounds are well absorbed at all GI PH and their absorption rate is usually higher than excretion); Class II- high permeability, low solubility (bioavailability of those products is limited by their solubility and rate of dissolution Class III - low permeability, high solubility (absorption is limited by the permeation rate but the drug is solvated very fast; if the formulation does not change the permeability or gastro-intestinal duration time, then class I criteria can be applied); Class IV - low permeability, low solubility (compounds have a poor bioavailability; usually they are not well absorbed over the intestinal mucosa and a high variability is expected).

The drugs are classified in BCS on the basis of following parameters: 1. Solubility; 2.Permeability; and 3. Rate of dissolution. Solubility class boundaries are based on the highest dose strength of an immediate release product. A drug is considered highly soluble when the highest dose strength is soluble in 250ml or less of aqueous media over the ph range of I to 7.5. The volume estimate of 250ml is derived from typical bioequivalence study protocols that prescribe administration of a drug product to fasting human volunteers with a glass of water. Permeability class boundaries are based indirectly on the extent of absorption of a drug substance in humans and directly on the measurement of rates of mass transfer across human intestinal membrane. Alternatively non-human systems capable of prediction the drug absorption systems capable of predicting the drug absorption in humans can be used (such as in-vitro culture methods). A drug substance is considered highly permeable when the extent of absorption in humans is determined to be 90% or more of the administered dose based on a mass-balance determination or in comparison to and intravenous dose. With respect to dissolution class boundaries, an immediate release products is considered rapidly dissolving when no less than 85% of the labeled amount of the drug substance dissolve within 15 minutes using USP Dissolution Apparatus 1 at 100 RPM or Apparatus 2 at 50 RPM in a volume of900ml or less in following media,) 0.1 N HCI or simulated gastric fluid or pH 4.5 buffer and pH 6.8 buffer or simulated intestinal fluid.

In some embodiments, the additional active ingredient is a drug including, but not limited to the following drugs: tripranavir, cefditoren pivoxil, tadalafil, mycophenolic acid, posaconazole, lapatinib, bromocriptine, ticagrelor, sorafetinib, itraconazole, erlotinib, sirolimus, alvimopan, naltrexone, vardenafil, rosuvastatin, maraviroc, ritonavir, efavirez, celecoxib, atovaquone, raloxifene, finasteride, everolimus, and dodrenarone.

In some embodiments, the additional active ingredient is selected from the groups consisting of antineoplastic, antifungal, antiviral, anticonvulsant, antiepileptic, antidepressant, immunosuppressant, anti-inflammatory and erectile dysfunction drugs.

In some embodiments, exemplary antineoplastic drugs suitable for use as an additional active ingredient include, but are not limited to: I) alkaloids, including microtubule inhibitors (e.g., vincristine, vinblastine, and vindesine, etc.), microtubule stabilizers (e.g., paclitaxel (TAXOL), and docetaxel, etc.), and chromatin function inhibitors, including topoisomerase inhibitors, such as epipodophyllotoxins (e.g., etoposide (VP-16), and teniposide (VM-26), etc.), and agents that target topoisomerase I (e.g., camptothecin and isirinotecan (CPTII), etc.); 2) covalent DNA-binding agents (alkylating agents), including nitrogen mustards (e.g., mechlorethamine, chlorambucil, cyclophosphamide, ifosphamide, and busulfan (MYLERAN), etc.), nitrosoureas (e.g., carmustine, lomustine, and semustine, etc.), and other alkylating agents (e.g., dacarbazine, hydroxymethylmelamine, thiotepa, and mitomycin, etc.); 3) noncovalent DNA-binding agents (antitumor antibiotics), including nucleic acid inhibitors (e.g., dactinomycin (actinomycin D), etc.), anthracyclines (e.g., daunorubicin (daunomycin, and cerubidine), doxorubicin (adriamycin), and idarubicin (idamycin), etc.), anthracenediones (e.g., anthracycline analogues, such as mitoxantrone, etc.), bleomycins (BLENOXANE), etc., and plicamycin (mithramycin), etc.; 4) antimetabolites, including antifolates (e.g., methotrexate, FOLEX, and MEXATE, etc.), purine antimetabolites (e.g., 6-mercaptopurine (6-MP, PURINETHOL), 6-thioguanine (6-TG), azathioprine, acyclovir, ganciclovir, chlorodeoxyadenosine, 2-chlorodeoxyadenosine (CdA), and 2'-deoxycoformycin (pentostatin), etc.), pyrimidine antagonists (e.g., fluoropyrimidines (e.g., 5-fluorouracil (ADRUCIL), 5-fluorodeoxyuridine (FdUrd) (floxuridine)) etc.), and cytosine arabinosides (e.g., CYTOSAR (ara-C) and fludarabine, etc.); 5) enzymes, including L-asparaginase, and hydroxyurea, etc.; and 6) platinum compounds (e.g., cisplatin and carboplatin, etc.).

In some embodiments, exemplary antifungal drugs suitable for use as an additional active ingredient include, but are not limited to nystatin, amphotericin B, griseofulvin, miconazole, ketoconazole, terbinafine, itraconazole, fluconazole, posaconazole, and voriconazole. In some embodiments, exemplary antiviral drugs suitable for use in dosage forms of the present invention include, but are not limited to abacavir, aciclovir, acyclovir, adefovir, amantadine, amprenavir, ampligen, arbidol, atazanavir, atripla, boceprevir, cidofovir, combivir, darunavir, delavirdine, didanosine, docosanol, edoxudine, efavirenz, emtricitabine, enfuvirtide, entecavir, famciclovir, fomivirsen, fosamprenavir, foscamet, fosfonet, ganciclovir, ibacitabine, imunovir, idoxuridine, imiquimod, indinavir, inosine, lamivudine, lopinavir, loviride, maraviroc, moroxydine, methisazone, nelfinavir, nevirapine, nexavir, oseltamivir (Tamiflu), peginterferon alfa-2a, penciclovir, peramivir, pleconaril, podophyllotoxin, raltegravir, ribavirin, rimantadine, ritonavir, pyramidine, saquinavir, stavudine, tea tree oil, tenofovir, tenofovir disoproxil, tipranavir, trifluridine, trizivir, tromantadine, truvada, valaciclovir (Valtrex), valganciclovir, vicriviroc, vidarabine, viramidine, zalcitabine, zanamivir (Relenza) and zidovudine.

In some embodiments, exemplary anticonvulsant drugs suitable for use as an additional active ingredient include, but are not limited to pregabalin, gabapentin, carbamazepine, and oxcarbazepine.

In some embodiments, exemplary antiepileptic and anticonvulsant drugs suitable for use as an additional active ingredient include, but are not limited to pregabalin, gabapentin, carbamazepine, and oxcarbazepine and alprazolam, bretazenil, bromazepam, brotizolam, chlordiazepoxide, cinolazepam, clonazepam, clorazepate, clotiazepam, cloxazolam, delorazepam, diazepam, estazolam, etizolam, flunitrazepam, flurazapam, flutoprazepam, halazepam, ketazolam, loprazolam, lorazepam, lormetazepam, medazepam, midazolam, nemetazepam, nitrazepam, nordazepam, oxazepam, phenazepam, pinazepaam, prazepam, premazepam, quazepam, temazepam, tetrazepam, triazolam, clobazam, DMCM, flumazenil, eszopiclone, zaleplon, zolpidem, and zopiclone.

In some embodiments, exemplary antidepressant drugs suitable for use as an additional active ingredient include, but are not limited to tricyclic compounds such as bupropion, nortriptyline, desipramine, amitriptyline, amitriptylinoxide, butriptyline, clomipramine, demexiptiline, dibenzepin, dimetacrine, dosulepin/dothiepin, doxepin, imipramine, amineptine, iprindole, opipramol, tianeptine, trimipramine, imipraminoxide, lofepramine, melitracin, metapramine, nitroxazepine, noxiptiline, pipofezine, propizepine, protriptyine, and quinupramine; SNRis such as duloxetine, venlafaxine, desvenlafaxine, milnacipran, levomilnacipran, sibutramine, bicifadine, and SEP-227162; and SSRis such as citalopram, dapoxetine, escitalopram, fluoxetine, fluvoxamine, indalpin, paroxetine, sertraline, and zimelidine.

In some embodiments, exemplary immunosuppressant drugs suitable for use as an additional active ingredient include, but are not limited to azathioprine, mycophenolic acid, leflunomide, teriflunomide, methotrexate, tacrolimus, cyclosporin, pimecrolimus, abetimus, gusperimus, thalidomide, lenalidomide, anakinra, sirolimus, everolimus, ridaforolimus, tesirolimus, umirolimus, and zotarolimus.

In some embodiments, exemplary erectile dysfunction drugs suitable for use as an additional active ingredient include, but are not limited to Tadalafil, Vardenafil, Sildenafil, Alprostadil, Papaverine, and Phentolamine.

In some embodiments, the additional active ingredient is a non-steroidal anti-inflammatory drugs (NSAIDS). The NSAIDS can, for example, be selected from the following: choline salicylate (Arthropan) celecoxib (Celebrex); diclofenac potassium (Cataflam); diclofenac sodium (Voltaren, Voltaren XR); diclofenac sodium with misoprostol (Arthrotec); diflunisal (Dolobid); etodolac (Lodine, Lodine XL); fenoprofen calcium (Nalfon); flurbiprofen (Ansaid); ibuprofen (Advil, Motrin, Motrin IB, Nuprin); indomethacin (Indocin, Indocin SR); ketoprofen (Actron, Orudis, Orudis KT, Oruvail); magnesium salicylate (Arthritab, Bayer Select, Doan's Pills, Magan, Mobidin, Mobogesic); meclofenamate sodium (Meclomen); mefenamic acid (Ponstel); meloxicam (Mobic); nabumetone (Relafen); naproxen (Naprosyn, Naprelan); naproxen sodium (Aleve, Anaprox); oxaprozin (Daypro); piroxicam (Feldene); rofecoxib (Vioxx); salsalate (Amigesic, Anaflex 750, Disalcid, Marthritic, Mono-Gesic, Salflex, Salsitab); sodium salicylate (various generics); sulindac (Clinoril); tolmetin sodium (Tolectin); and valdecoxib (Bextra).

### 2. Powders

Another aspect of the present invention relates to powders prepared by the methods described above. In some embodiments, the dry powders of the present invention are tabletable powders. In some embodiments, the dry powders of the present invention can be tableted using standard tableting equipment producing more than 10,000 tablets per hour and the tablets can be prepared continuously for hours.

The present powder is prepared from the aqueous mixture by removing the water. The process for preparation of dry powder include various state of the art methods within pharmaceutical production like drying at increased temperature, vacuum drying, freeze drying, spray drying and spray granulation. Spray drying/spray granulation methods are the most preferred methods for preparation of tablettable powder.

In some embodiments, the powders of the present invention comprise an oil component and beta-cyclodextrin in a defined ratio which may preferably be expressed as a weight/weight (w/w) percentage of the oil component in the powder. The powders of the present invention therefore comprise from 10% to 40%, 20% to 40%, 30% to 40%, 10% to 35%, 20% to 35%, 25% to 35%, 30% to 35%, at least 10%, at least 20%, at 30% or at least 35% w/w of an oil component, wherein w/w refers to the total weight of the oil component to the total weight of the powder.

In some embodiments, one or more of these omega 3 fatty acids and derivatives thereof are the main omega-3 components in the dry powder. In some embodiments, one or more of these omega 3 fatty acids and derivatives thereof are the main oil components in the dry powder (e.g., the oil component in the powder comprises greater than about 70%, 80%, w/w of the specified omega-3 fatty acid or derivative thereof wherein w/w refers to the total weight of the specified omega-3 fatty acid or derivative thereof per the total weight of the oil component). In some embodiments, one or more of these omega 3 fatty acids and derivatives thereof are from a marine source, such as fish, algae, or have been prepared from raw products from fish or algae. In some embodiments, one or more of these omega 3 fatty acids and derivatives thereof are from plants or vegetables or have been prepared from raw products from plants and vegetables. In some embodiments, the lipid component preferably comprises from about 30% to 60% w/w EPA and/or DHA. In some embodiments, the lipid component, and thus the powders, may comprise an additional active ingredient as described in detail above.

In some embodiments, the omega-3 fatty acids or derivatives are preferably selected from EPA and DHA and combinations thereof. In some embodiments, the omega 3 fatty acids and derivatives thereof comprise more EPA than DHA (i.e., the ratio of EPA: DHA is greater than 1:1). In some embodiments, the omega 3 fatty acids and derivatives thereof comprise more DHA than EPA (i.e., the ratio of DHA: EPA is greater than 1:1). In some embodiments, the omega 3 fatty acids and derivatives thereof are enriched for EPA, e.g., more than 90% w/w of the total omega 3 fatty acids and derivatives thereof in the powder is EPA where the w/w is the weight of EPA per total weight of fatty acids in the powder. In some embodiments, the omega 3 fatty acids and derivatives thereof are enriched for DHA, e.g., more than 90% w/w of the total omega 3 fatty acids and derivatives thereof in the powder is DHA where the w/w% is the weight of DHA per total weight of fatty acids in the powder.

In some embodiments, the powders may be further characterized according to their total omega-3 content. In some embodiments, the dry powders of the present invention comprise more than 10% w/w, more than 15% w/w, more than 20% w/w, more than 25% w/w or more than 30% w/w of omega-3 triglycerides.

### 3) Tablets

Another aspect of the present invention relates to tablets formed from the powders described above. As described above, in some embodiments, the dry powders of the present invention are tabletable powders. In some embodiments, the dry powders of the present invention can be tableted using standard tableting equipment producing more than 10,000 tablets per hour and the tablets can be prepared continuously for hours. In some preferred embodiments, the tablets have a crushing strength of greater than 3, 4, 5, 6, 7, 8, 9, 10, 11, 12, 13, 14 or 15 kN, or from about 3 to 50, 3 to 40, 3 to 30, 3 to 20, 5 to 50, 5 to 40, 5 to 30, 5 to 20, 5 to 10, 7 to 50, 7 to 40, 7 to 30, 7 to 20, 10 to 50, 10 to 40, 10 to 30, or 10 to 20 kN.

In some embodiments, the tablets of the present invention comprise an oil component and beta-cyclodextrin in a defined ratio which may preferably be expressed as a weight/weight (w/w) percentage of the oil component in the powder.

The tablets of the present invention therefore comprise from 10% to 40%, 20% to 40%, 30% to 40%, 10% to 35%, 20% to 35%, 25% to 35%, 30% to 35%, at least 10%, at least 20%, at 30% or at least 35% w/w of an oil component, wherein w/w refers to the total weight of the oil component to the total weight of the tablet.

In some embodiments, one or more of these omega 3 fatty acids and derivatives thereof are the main omega-3 components used to prepare the dry powder. In some embodiments, one or more of these omega 3 fatty acids and derivatives thereof are the main omega-3 components in the dry powder. In some embodiments, one or more of these omega 3 fatty acids and derivatives thereof are the main oil components in the dry powder (e.g., the oil component in the powder comprises greater than about 70%, 80%, w/w of the specified omega-3 fatty acid or derivative thereof wherein w/w refers to the total weight of the specified omega-3 fatty acid or derivative thereof per the total weight of the oil component). In some embodiments, one or more of these omega 3 fatty acids and derivatives thereof are from a marine source, such as fish, algae, or have been prepared from raw products from fish or algae. In some embodiments, one or more of these omega 3 fatty acids and derivatives thereof are from plants or vegetables or have been prepared from raw products from plants and vegetables. In some embodiments, the lipid component used to prepare the powder and thus the tablets may comprise an additional active ingredient as described in detail above.

In some embodiments, the omega-3 fatty acids or derivatives are preferably selected from EPA and DHA and combinations thereof. In some embodiments, the omega 3 fatty acids and derivatives thereof comprise more EPA than DHA (i.e., the ratio of EPA: DHA is greater than 1:1). In some embodiments, the omega 3 fatty acids and derivatives thereof comprise more DHA than EPA (i.e., the ratio of DHA: EPA is greater than 1:1). In some embodiments, the omega 3 fatty acids and derivatives thereof are enriched for EPA, e.g., more than 90% w/w of the total omega 3 fatty acids and derivatives thereof in the powder is EPA where the w/w is the weight of EPA per total weight of fatty acids in the powder. In some embodiments, the omega 3 fatty acids and derivatives thereof are enriched for DHA, e.g., more than 90% w/w of the total omega 3 fatty acids and derivatives thereof in the powder is DHA where the w/w% is the weight of DHA per total weight of fatty acids in the powder. In some embodiments, the lipid component preferably comprises from about 30% to 60% w/w EPA and/or DHA.

In some embodiments, the tablets may be further characterized according to their total omega-3 content. The tablets of the present invention comprise more than 10% w/w, more than 15% w/w, more than 20% w/w, more than 25% w/w or more than 30% w/w of omega-3 triglycerides where w/w refers to the total weight of the omega-3 triglycerides per the total weight of the tablet.

Accordingly, in some embodiments, the tablets of the present invention comprise a surfactant in a defined ratio to the amount of the oil component in the tablet, from 10% to 20% w/w, 10% to 30% w/w, 15% to 30% w/w, 18% to 30% w/w, 20% to 30% w/w, or 10% to 35% w/w surfactant or combination of surfactants, wherein w/w refers to the total weight of surfactant (or combination thereof) to the total weight of the oil component including the surfactant.

The surfactants in tablets are diglycerides and the most preferred surfactants are diglycerides where one or more of the acids are omega-3 fatty acids. The typically most preferred diglycerides comprise of a mixture of diglyceride compounds where the fatty acid components in the mixture of diglyceride molecules can be saturated, monounsaturated and/or polyunsaturated, including omega-3 fatty acids like EPA and DHA.

In some embodiments, the tablets are coated. Suitable coatings include, but are not limited to, polyvinyl acetate, methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, cellulose acetate trimellitate, and sodium alginate.

### EXAMPLES

### Example 1. Preparation of 30:70 triglyceride:beta-cyclodextrin complexes with diglycerides.

Beta-cyclodextrin (1000g) was suspended in water at room temperature. A mixture of EPA and DHA (60% w/w) and various fatty acids as triglycerides, diglycerides and monoglycerides (430 g) was added. The mixture was stirred for 1 hour. The water was evaporated. The product was off-white, tabletable powder.

### Example 2. Preparation of 30:70 triglyceride:beta-cyclodextrin complexes prepared

**with 7% diglycerides (not according to the claimed invention)**

The product was prepared as in example 1 with oil with glyceride composition (triglycerides/diglycerides/monoglycerides) as 91/7/1 area %. The product was a slurry with obvious oil layers, it was not possible to prepare dry powders from this mixture.

### Example 3. Preparation of 30:70 triglyceride:beta-cyclodextrin complexes prepared with 19 area % diglycerides.

The product was prepared as in example 1 with oil with glyceride composition (triglycerides/diglycerides/monoglycerides) as 80/19/1 area %.

The product was dry, off-white to yellow powder. The powder was tabletable.

### Example 4. Preparation of 30:70 triglyceride:beta-cyclodextrin complexes prepared with 24 area % diglycerides.

The product was prepared as in example 1 with oil with glyceride composition (triglycerides/diglycerides/monoglycerides) as 74/25/1 area %.

The product was dry, off-white powder. The powder was directly tabletable.

### Example 5. Preparation of 30:70 triglyceride:beta-cyclodextrin complexes prepared with 32 area % diglycerides.

The product was prepared as in example 1 with oil with glyceride composition (triglycerides/diglycerides/monoglycerides) as 67/32/1 area %.

The product was dry, off-white powder. The powder was directly tabletable.

### Example 6. Preparation of 30:70 triglyceride:beta-cyclodextrin complexes prepared with 34 area % diglycerides.

The product was prepared as in example 1 with oil with glyceride composition (triglycerides/diglycerides/monoglycerides) as 65/34/1 area %.

The product was dry, off-white powder. The powder was directly tabletable.

### Example 7. Tablets prepared from 30:70 triglyceride:beta-cyclodextrin complexes prepared with 27 area % diglycerides.

Tablets comprising 96% (w/w) triglyceride:beta-cyclodextrin complexes prepared from oil with glyceride composition (triglycerides/diglycerides/monoglycerides) as 74/25/1 area % was prepared in a conventional tableting machine.

The tablets achieved a crushing strength of 9.1 kN.

### Example 8. Preparation of triglyceride:beta-cyclodextrin powder with spray granulation

The powder was prepared as in example 1, the method for water evaporation was spray granulation. The powder comprised of rounded particles with particle size distribution between 50-650 microns. The powder was directly tablettable.

### Example 9. Preparation of 30:70 triglyceride:beta-cyclodextrin complexes with different content of diglyceride (DG) optionally added a surfactant.

The powder products were prepared as in example 1, spray granulated when complexes were achieved and tableted.

### Example 10. Results for powder and tableting experiments.

The following Table provides results from experiments in which varying levels of surfactants, including diglycerides and other added surfactants, were used in powder formulations.

The first 10 experiments demonstrate that triglyceride omega-3 oil with a low content of diglycerides (8%) with added surfactants (1% or 10%) (not according to the claimed invention) did not result in a powder for direct compaction (DC).

The next experiment shows that triglyceride omega-3 oil with relative high content of diglycerides (27%) result in powder for direct compaction. The next 3 experiments show that addition of surfactants to the composition results in a minor reduction of the crushing strength of the tablets, however, the powders were still tablettable.

The final 3 experiments have been performed with three different ethyl ester oils (not according to the claimed invention).

EE60 comprise 60 % omega-3, EE5325 comprise 33% EPA and 23%DHA and EE4020 comprise 40% EPA and 20% DHA. The results show that all ethyl ester oil composition tested, pain and with surfactants(s) formed powder that were directly compactable.

A control tablet experiment using a triglyceride omega-3 comprising 69% diglyceride (not according to the claimed invention) showed lower crushing strength than comparative tablets comprising appr. 30% diglyceride (same oil loading in both tablets).

| **Oil** | | | **Surfactants** | | | **Powder** | **Tablets** | |
|---|---|---|---|---|---|---|---|---|
| ***Raw material*** | ***Oil load*** | ***MG*/*DG* /*TG*** | ***Surfactant*** | ***HLB*** | ***Amount*** | | ***Crushing strength (kN)*** | ***Friability (%)*** |
| TG3322 | 30% | 1/8/91 | Span 85 | 1.8 | 1% | No | - | - |
| | | | Tween 40 | 15.6 | 1% | No | - | - |
| | | | Tween 40+Span 85 | 9.2 | 1% | No | - | - |
| | | | Span 20 | 8.6 | 1% | No | - | - |
| | | | Span 80 | 4.3 | 1% | No | - | - |
| | | | Tween 80 | 15 | 1% | No | - | - |
| | | | Tween 60 | 14.9 | 1% | No | - | - |
| | | | Span 85 | 1.8 | 10% | No | - | - |
| | | | Tween 40 | 15.6 | 10% | No | - | - |
| | | | Span 20 + Tween 40 | 12.1 | 10% | No | - | - |
| | | | | | | | | |

| **Oil** | | | **Surfactants** | | | **Powder** | **Tablets** | |
|---|---|---|---|---|---|---|---|---|
| ***Raw material*** | ***Oil load*** | ***MG*/*DG* /*TG*** | ***Surfactant*** | ***HLB*** | ***Amount*** | | ***Crushing strength (kN)*** | ***Friability (%)*** |
| TG3322 | 30% | 1/27/69 | None | - | - | DC grade | 9.1 | 99.9 |
| | | | Span 85 | 1.8 | 1% | DC grade | 8.7 | 99.9 |
| | | | Tween 40 | 15.6 | 0.1% | DC grade | 7.6 | 99.9 |
| | | | Tween 40+Span 85 | 9.2 | 1%+1% | DC grade | 7.6 | 99.9 |
| | | | | | | | | |

| **Oil** | | | **Surfactants** | | | **Powder** | **Tablets** | |
|---|---|---|---|---|---|---|---|---|
| ***Raw material*** | ***Oil load*** | ***MG*/*DG* /*TG*** | ***Surfactant*** | ***HLB*** | ***Amount*** | | ***Crushing strength (kN)*** | ***Friability (%)*** |
| EE60 | 30% | - | None | - | - | DC grade | 8.6 | 99.9 |
| EE3525 | 30% | - | Tween 40 + Span 85 | 9.2 | 1%+1% | grade | 5.4 | 99.8 |
| EE4020 | 30% | - | Span 85 | 1.8 | 0.5% | DC grade | 6.9 | 99.9 |

## Claims

1. A composition comprising:
a dry powder comprising beta-cyclodextrin in an amount of from 60% to 90% w/w of said powder and a lipid component in an amount of from about 10% to 40% w/w of said powder, wherein said lipid component comprises an omega-3 fatty acid or derivative thereof selected from the group consisting of omega-3 triglycerides, wherein said omega-3 fatty acid or derivative thereof selected from the group consisting of omega-3 triglycerides comprises EPA and DHA fatty acids at a concentration of from 10% to 99% w/w of the fatty acids in said omega-3 triglycerides, wherein said lipid component is characterized as having a surfactant content of from about 10% to 35% w/w of said lipid component, wherein said surfactant is a diglyceride of fatty acids.

2. The composition of claim 1, wherein said omega-3 fatty acid or derivative thereof selected from the group consisting of omega-3 triglycerides comprises EPA and DHA fatty acids at a concentration of from 10% to 70% w/w of the fatty acids in said omega-3 triglycerides.

3. The composition of claim 1, wherein said omega-3 fatty acid or derivative thereof selected from the group consisting of omega-3 triglycerides comprises EPA and DHA fatty acids at a concentration of from 30% to 60% w/w of the fatty acids in said omega-3 triglycerides.

4. The composition of claim 1, wherein said omega-3 triglycerides are a marine oil, preferably wherein said marine oil is selected from the group consisting of fish oil, squid oil and algal oil.

5. The composition according to any one of claims 1 to 4, wherein said diglycerides of fatty acids comprise a mixture of diglyceride compounds wherein the fatty acid components of the diglycerides compounds are selected from polyunsaturated fatty acids.

6. The composition according to claim 5, wherein said polyunsaturated fatty acids are omega-3 fatty acids,
wherein said omega-3 fatty acids are selected from EPA and DHA.

7. A tableted lipid formulation comprising beta-cyclodextrin in a concentration of from 60% to 90% w/w of said tablet and a lipid component in a concentration of from 10% to 40% w/w of said tablet, wherein said lipid component comprises an omega-3 fatty acid or derivative thereof selected from the group consisting of omega-3 triglycerides, wherein said omega-3 fatty acid or derivative thereof selected from the group consisting of omega-3 triglycerides, comprises EPA and DHA fatty acids at a concentration of from 10% to 99% w/w of the fatty acids in said omega-3 triglycerides, wherein said lipid component is characterized as having a diglyceride content of from 10% to 35% w/w of said lipid component.

8. The tableted lipid formulation of claim 7, wherein said omega-3 fatty acid or derivative thereof selected from the group consisting of omega-3 triglycerides comprises EPA and DHA fatty acids at a concentration of from 10% to 70% w/w of the fatty acids in said omega-3 triglycerides.

9. The tableted lipid formulation of claim 7, wherein said omega-3 fatty acid or derivative thereof selected from the group consisting of omega-3 triglycerides comprises EPA and DHA fatty acids at a concentration of from 30% to 60% w/w of the fatty acids in said omega-3 triglycerides.

10. The tableted lipid formulation of claim 7, wherein said omega-3 triglycerides are a marine oil, preferably wherein said marine oil is selected from the group consisting of fish oil, squid oil and algal oil.

11. The tableted lipid formulation of claim 7, wherein said diglycerides of fatty acids comprise a mixture of diglyceride compounds wherein the fatty acid components of the diglycerides compounds are selected from polyunsaturated fatty acids.

12. The tableted lipid formulation of claim 11, wherein said polyunsaturated fatty acids are omega-3 fatty acids, wherein said omega-3 fatty acids are selected from EPA and DHA.

13. The tableted lipid formulation according to any one of claims 7 to 12, wherein the tableted formulation is coated, preferably wherein the tableted formulation is coated with an agent selected from the group consisting of polyvinyl acetate, methyl acrylate-methacrylic acid copolymers, cellulose acetate phthalate (CAP), cellulose acetate succinate, hydroxypropyl methyl cellulose phthalate, hydroxypropyl methyl cellulose acetate succinate (hypromellose acetate succinate), polyvinyl acetate phthalate (PVAP), methyl methacrylate-methacrylic acid copolymers, cellulose acetate trimellitate, and sodium alginate.

14. A process for making a tabletable lipid powder comprising:
combining an aqueous solution of beta-cyclodextrin with a lipid component in an amount of from 10% to 40% w/w of said beta-cyclodextrin said solution, wherein said lipid component comprises an omega-3 fatty acid or derivative thereof selected from the group consisting of omega-3 triglycerides, wherein said omega-3 fatty acid or derivative thereof selected from the group consisting of omega-3 triglycerides comprises EPA and DHA fatty acids at a concentration of from 10% to 99% w/ of the fatty acids in said omega-3 triglycerides, wherein said lipid component comprises one or more surfactants at a concentration of from 10% to 35% w/w of said lipid component, wherein said surfactant is a diglyceride of fatty acids;
mixing said aqueous solution of beta-cyclodextrin and said lipid component to provide a mixture; and
removing water from said mixture to provide a dry powder, preferably by spray granulating.

## Patentansprüche

1. Zusammensetzung, umfassend:
ein trockenes Pulver, umfassend beta-Cyclodextrin in einer Menge von 60 % bis 90 % w/w des Pulvers und eine Lipidkomponente in einer Menge von etwa 10 % bis 40 % w/w des Pulvers, wobei die Lipidkomponente eine Omega-3-Fettsäure oder ein Derivat davon aus der Gruppe bestehend aus Omega-3-Triglyceriden umfasst, wobei die Omega-3-Fettsäure oder das Derivat davon aus der Gruppe bestehend aus Omega-3-Triglyceriden EPA- und DHA-Fettsäuren in einer Konzentration von 10 % bis 99 % w/w der Fettsäuren in den Omega-3-Triglyceriden umfasst, wobei die Lipidkomponente **dadurch gekennzeichnet ist, dass** sie einen Tensidgehalt von etwa 10 % bis 35 % w/w der Lipidkomponente aufweist, wobei es sich bei dem Tensid um ein Diglycerid von Fettsäuren handelt.

2. Zusammensetzung nach Anspruch 1, wobei die Omega-3-Fettsäure oder das Derivat davon aus der Gruppe bestehend aus Omega-3-Triglyceriden EPA-und DHA-Fettsäuren in einer Konzentration von 10 % bis 70 % w/w der Fettsäuren in den Omega-3-Triglyceriden umfasst.

3. Zusammensetzung nach Anspruch 1, wobei die Omega-3-Fettsäure oder das Derivat davon aus der Gruppe bestehend aus Omega-3-Triglyceriden EPA-und DHA-Fettsäuren in einer Konzentration von 30 % bis 60 % w/w der Fettsäuren in den Omega-3-Triglyceriden umfasst.

4. Zusammensetzung nach Anspruch 1, wobei es sich bei den Omega-3-Triglyceriden um ein marines Öl handelt, wobei das marine Öl aus der Gruppe bestehend aus Fischöl, Tintenfischöl und Algenöl ausgewählt ist.

5. Zusammensetzung nach einem der Ansprüche 1 bis 4, wobei die Diglyceride von Fettsäuren ein Gemisch von Diglyceridverbindungen umfasst, wobei die Fettsäurekomponenten der Diglyceridverbindungen aus mehrfach ungesättigten Fettsäuren ausgewählt sind.

6. Zusammensetzung nach Anspruch 5, wobei es sich bei den mehrfach ungesättigten Fettsäuren um Omega-3-Fettsäuren handelt,
wobei die Omega-3-Fettsäuren aus EPA und DHA ausgewählt sind.

7. Tablettierte Lipidformulierung, umfassend beta-Cyclodextrin in einer Konzentration von 60 % bis 90 % w/w der Tablette und eine Lipidkomponente in einer Konzentration von 10 % bis 40 % w/w der Tablette, wobei die Lipidkomponente eine Omega-3-Fettsäure oder ein Derivat davon aus der Gruppe bestehend aus Omega-3-Triglyceriden, wobei die Omega-3-Fettsäure oder das Derivat davon aus der Gruppe bestehend aus Omega-3-Triglyceriden EPA- und DHA-Fettsäuren in einer Konzentration von 10 % bis 99 % w/w der Fettsäuren in den Omega-3-Triglyceriden umfasst, wobei die Lipidkomponente **dadurch gekennzeichnet ist, dass** sie einen Diglyceridgehalt von 10 % bis 35 % w/w der Lipidkomponente aufweist.

8. Tablettierte Lipidformulierung nach Anspruch 7, wobei die Omega-3-Fettsäure oder das Derivat davon aus der Gruppe bestehend aus Omega-3-Triglyceriden EPA- und DHA-Fettsäuren in einer Konzentration von 10 % bis 70 % w/w der Fettsäuren in den Omega-3-Triglyceriden umfasst.

9. Tablettierte Lipidformulierung nach Anspruch 7, wobei die Omega-3-Fettsäure oder das Derivat davon aus der Gruppe bestehend aus Omega-3-Triglyceriden EPA- und DHA-Fettsäuren in einer Konzentration von 30 % bis 60 % w/w der Fettsäuren in den Omega-3-Triglyceriden umfasst.

10. Tablettierte Lipidformulierung nach Anspruch 7, wobei es sich bei den Omega-3-Triglyceriden um ein marines Öl handelt, wobei das marine Öl vorzugsweise aus der Gruppe bestehend aus Fischöl, Tintenfischöl und Algenöl ausgewählt ist.

11. Tablettierte Lipidformulierung nach Anspruch 7, wobei die Diglyceride von Fettsäuren ein Gemisch von Diglyceridverbindungen umfasst, wobei die Fettsäurekomponenten der Diglyceridverbindungen aus mehrfach ungesättigten Fettsäuren ausgewählt sind.

12. Tablettierte Lipidformulierung nach Anspruch 11, wobei es sich bei den mehrfach ungesättigten Fettsäuren um Omega-3-Fettsäuren handelt, wobei die Omega-3-Fettsäuren aus EPA und DHA ausgewählt sind.

13. Tablettierte Lipidformulierung nach einem der Ansprüche 7 bis 12, wobei die tablettierte Formulierung überzogen ist, vorzugsweise wobei die tablettierte Formulierung mit einem Mittel aus der Gruppe bestehend aus Polyvinylacetat, Methylacrylat-Methacrylsäure-Copolymeren, Celluloseacetatphthalat (CAP), Celluloseacetatsuccinat, Hydroxypropylmethylcellulosephthalat, Hydroxypropylmethylcelluloseacetatsuccinat (Hypromelloseacetatsuccinat), Polyvinylacetatphthalat (PVAP), Methylmethacrylat-Methacrylsäure-Copolymeren, Celluloseacetattrimellitat und Natriumalginat überzogen ist.

14. Verfahren zur Herstellung eines tablettierbaren Lipidpulvers, umfassend:
das Vereinigen einer wässrigen Lösung von beta-Cyclodextrin mit einer Lipidkomponente in einer Menge von 10 % bis 40 % w/w des beta-Cyclodextrins der Lösung, wobei die Lipidkomponente eine Omega-3-Fettsäure oder ein Derivat davon aus der Gruppe bestehend aus Omega-3-Triglyceriden umfasst, wobei die Omega-3-Fettsäure oder das Derivat davon aus der Gruppe bestehend aus Omega-3-Triglyceriden EPA- und DHA-Fettsäuren in einer Konzentration von 10 % bis 99 % w/w der Fettsäuren in den Omega-3-Triglyceriden umfasst, wobei die Lipidkomponente ein oder mehrere Tenside in einer Konzentration von 10 % bis 35 % w/w der Lipidkomponente aufweist, wobei es sich bei dem Tensid um ein Diglycerid von Fettsäuren handelt;
das Mischen der wässrigen Lösung von beta-Cyclodextrin und der Lipidkomponente zur Bereitstellung einer Mischung und
das Entfernen von Wasser aus der Mischung zur Bereitstellung eines trockenen Pulvers, vorzugsweise durch Sprühgranulation.

## Revendications

1. Composition comprenant :
une poudre sèche comprenant de la bêta-cyclodextrine en une quantité comprise entre 60% et 90% p/p de ladite poudre et un composant lipidique en une quantité comprise entre environ 10% et 40% p/p de ladite poudre, où ledit composant lipidique comprend un acide gras oméga-3 ou un dérivé de celui-ci choisi dans le groupe constitué de triglycérides oméga-3, où ledit acide gras oméga-3 ou dérivé de celui-ci choisi dans le groupe constitué de triglycérides oméga-3 comprend les acides gras EPA et DHA à une concentration comprise entre 10% et 99% p/p des acides gras dans lesdits triglycérides oméga-3, où ledit composant lipidique est caractérisé comme ayant une teneur en tensioactif comprise entre environ 10% et 35% p/p dudit composant lipidique, où ledit tensioactif est un diglycéride d'acides gras.

2. Composition de la revendication 1, dans laquelle ledit acide gras oméga-3 ou dérivé de celui-ci choisi dans le groupe constitué de triglycérides oméga-3 comprend les acides gras EPA et DHA à une concentration comprise entre 10% et 70% p/p des acides gras dans lesdits triglycérides oméga-3.

3. Composition de la revendication 1, dans laquelle ledit acide gras oméga-3 ou dérivé de celui-ci choisi dans le groupe constitué de triglycérides oméga-3 comprend les acides gras EPA et DHA à une concentration comprise entre 30% et 60% p/p des acides gras dans lesdits triglycérides oméga-3.

4. Composition de la revendication 1, dans laquelle lesdits triglycérides oméga-3 sont une huile d'origine marine, dans laquelle de préférence ladite huile d'origine marine est choisie dans le groupe constitué d'huile de poisson, d'huile de calmar et d'huile d'algue.

5. Composition selon l'une quelconque des revendications 1 à 4, dans laquelle lesdits diglycérides d'acides gras comprennent un mélange de composés diglycérides où les composants acides gras des composés diglycérides sont choisis parmi des acides gras polyinsaturés.

6. Composition selon la revendication 5, dans laquelle lesdits acides gras polyinsaturés sont des acides gras oméga-3,
dans laquelle lesdits acides gras oméga-3 sont choisis parmi l'EPA et le DHA.

7. Formulation lipidique en comprimés comprenant de la bêta-cyclodextrine à une concentration comprise entre 60% et 90% p/p dudit comprimé et un composant lipidique à une concentration comprise entre 10% et 40% p/p dudit comprimé, où ledit composant lipidique comprend un acide gras oméga-3 ou un dérivé de celui-ci choisi dans le groupe constitué de triglycérides oméga-3, où ledit acide gras oméga-3 ou dérivé de celui-ci choisi dans le groupe constitué de triglycérides oméga-3, comprend les acides gras EPA et DHA à une concentration comprise entre 10% et 99% p/p des acides gras dans lesdits triglycérides oméga-3, où ledit composant lipidique est caractérisé comme ayant une teneur en diglycérides comprise entre 10% et 35% p/p dudit composant lipidique.

8. Formulation lipidique en comprimés de la revendication 7, dans laquelle ledit acide gras oméga-3 ou dérivé de celui-ci choisi dans le groupe constitué de triglycérides oméga-3 comprend les acides gras EPA et DHA à une concentration comprise entre 10% et 70% p/p des acides gras dans lesdits triglycérides oméga-3.

9. Formulation lipidique en comprimés de la revendication 7, dans laquelle ledit acide gras oméga-3 ou dérivé de celui-ci choisi dans le groupe constitué de triglycérides oméga-3 comprend les acides gras EPA et DHA à une concentration comprise entre 30% et 60% p/p des acides gras dans lesdits triglycérides oméga-3.

10. Formulation lipidique en comprimés de la revendication 7, dans laquelle lesdits triglycérides oméga-3 sont une huile d'origine marine, dans laquelle de préférence ladite huile d'origine marine est choisie dans le groupe constitué d'huile de poisson, d'huile de calmar et d'huile d'algue.

11. Formulation lipidique en comprimés de la revendication 7, dans laquelle lesdits diglycérides d'acides gras comprennent un mélange de composés diglycérides où les composants acides gras des composés diglycérides sont choisis parmi des acides gras polyinsaturés.

12. Formulation lipidique en comprimés de la revendication 11, dans laquelle lesdits acides gras polyinsaturés sont des acides gras oméga-3, où lesdits acides gras oméga-3 sont choisis parmi l'EPA et le DHA.

13. Formulation lipidique en comprimés selon l'une quelconque des revendications 7 à 12, dans laquelle la formulation en comprimés est enrobée, dans laquelle de préférence la formulation en comprimés est enrobée d'un agent choisi dans le groupe constitué d'acétate de polyvinyle, de copolymères d'acrylate de méthyle et d'acide méthacrylique, d'acétate phtalate de cellulose (CAP), d'acétate succinate de cellulose, de phtalate d'hydroxypropylméthylcellulose, d'acétate succinate d'hydroxypropylméthylcellulose (acétate succinate d'hypromellose), d'acétate phtalate de polyvinyle (PVAP), de copolymères de méthacrylate de méthyle et d'acide méthacrylique, de trimellitate d'acétate de cellulose et d'alginate de sodium.

14. Procédé de fabrication d'une poudre lipidique en comprimés comprenant :
la combinaison d'une solution aqueuse de bêta-cyclodextrine avec un composant lipidique en une quantité comprise entre 10% et 40% p/p de ladite solution de bêta-cyclodextrine, où ledit composant lipidique comprend un acide gras oméga-3 ou un dérivé de celui-ci choisi dans le groupe constitué de triglycérides oméga-3, où ledit acide gras oméga-3 ou dérivé de celui-ci choisi dans le groupe constitué de triglycérides oméga-3 comprend les acides gras EPA et DHA à une concentration comprise entre 10% et 99% p/p des acides gras dans lesdits triglycérides oméga-3, où ledit composant lipidique comprend un ou plusieurs tensioactif(s) à une concentration comprise entre 10% et 35% p/p dudit composant lipidique, où ledit tensioactif est un diglycéride d'acides gras ;
le mélange de ladite solution aqueuse de bêta-cyclodextrine et dudit composant lipidique pour fournir un mélange ; et
l'élimination de l'eau dudit mélange pour fournir une poudre sèche, de préférence par granulation par pulvérisation.
